# EUROPÄISCHE PATENTANMELDUNG

(11) **EP 1 129 682 A2**
(43) Veröffentlichungstag der Anmeldung: **05.09.2001**
(21) Anmeldenummer: 01104773.5
(22) Anmeldetag: 27.02.2001
(51) Int. Cl.: A61J 15/00, A61M 39/22

(54) **Vorrichtung zur Flüssigkeitsbilanzierung bei enteral ernährten Patienten**

(30) Priorität: 01.03.2000 DE 10009806; 26.05.2000 DE 10026120
(71) Anmelder: Krütten, Viktor, D-65510 Idstein (DE)
(72) Erfinder: Krütten, Viktor, D-65510 Idstein (DE)
(74) Vertreter: Müller, Eckhard, Dr.

(57) **Zusammenfassung**

Es wird eine Vorrichtung (10) zur Flüssigkeitsbilanzierung bei enteral ernährten Patienten mit einem ersten Behälter (12) und einem zweiten Behälter (14) beschrieben, die jeweils mit einer zum Patienten führenden Anschlußleitung (16) über ein Anschlußstück (18) in Verbindung stehen, wobei die Behälter (12, 14) unterschiedliche Inhalte aufweisen können. Das Anschlußstück (18) ist als Absperrverteilervorrichtung (20) mit mehreren Funktionsstellungen ausgebildet, wobei mit der Absperrverteilervorrichtung (20) die Strömungsverbindung zwischen dem ersten und/oder zweiten Behälter (12, 14) und der Anschlußleitung (16) wahlweise hergestellt und unterbrochen wird (Figur 1).

## Beschreibung

Die Erfindung betrifft eine Vorrichtung zur Flüssigkeitsbilanzierung bei enteral ernährten Patienten mit einem ersten Behälter und einem zweiten Behälter, die jeweils mit einer zum Patienten führenden Anschlußleitung über ein Anschlußstück in Verbindung stehen, wobei die Behälter unterschiedliche Inhalte, wie Nahrung und Flüssigkeit, unterschiedliche Flüssigkeiten beziehungsweise Nahrungen oder dergleichen enthalten.

Die bislang eingesetzten Vorrichtungen zur Flüssigkeitsbilanzierung bei enteral ernährten Patienten weisen den Nachteil einer komplizierten Handhabung auf. Die beiden Behälter werden jeweils über separate Zufuhrleitungen mit einem Anschlußstück verbunden, wobei dann vom Anschlußstück eine Anschlußleitung hin zum Patienten geführt ist. In jeder der Zufuhrleitungen zum Anschlußstück ist eine Dosiervorrichtung vorgesehen, so daß zum einen jede der Dosiervorrichtungen separat eingestellt werden muß. Zum anderen ist es bei den bekannten Vorrichtungen erforderlich, bei dem Anschluß eines Behälters die jeweilige Zufuhrleitung zu diskonektieren. Insgesamt lassen sich die bekannten Vorrichtungen nur umständlich und aufwendig bedienen.

Demgegenüber liegt der Erfindung die Aufgabe zugrunde, eine Vorrichtung mit den eingangs genannten Merkmalen dahingehend weiterzubilden, daß eine einfache und rasche Handhabung ermöglicht ist.

Diese Aufgabe wird bei der Vorrichtung mit den eingangs genannten Merkmalen im wesentlichen dadurch gelöst, daß das Anschlußstück als Absperrverteilervorrichtung mit mehreren Funktionsstellungen ausgebildet ist, mit der die Strömungsverbindung zwischen dem ersten und/oder zweiten Behälter und der Anschlußleitung wahlweise hergestellt und unterbrochen wird

Aufgrund dieser Maßnahme ist es nicht mehr erforderlich, die Zufuhrleitung zu dem jeweiligen Behälter zu diskonektieren, um diesen Behälter anzuschließen. Weiterhin kann aufgrund dieser Maßnahme die Vorrichtung unter Einsatz einer einzigen Dosiervorrichtung betrieben werden, wodurch der Handhabungsbeziehungsweise Einstellungsaufwand weiter reduziert wird.

Nach einer ersten vorteilhaften Weiterbildung der Erfindung weist die Absperrverteilervorrichtung drei Funktionsstellungen auf, wobei in einer ersten Funktionsstellung nur der erste Behälter in Strömungsverbindung und in einer zweiten Funktionsstellung nur der zweite Behälter in Strömungsverbindung mit der Anschlußleitung stehen und in einer dritten Funktionsstellung die Strömungsverbindung von erstem und zweiten Behälter zur Anschlußleitung unterbrochen ist. Aufgrund dieser Maßnahme kann der Inhalt der Behälter durch entsprechende Einstellung der Absperrverteilervorrichtung dem Patienten über die Anschlußleitung alternativ zugeführt werden, wobei jedoch auch die Möglichkeit gegeben ist, die Zufuhr des Behälterinhaltes aus beiden Behältern zu unterbinden.

Die Absperrverteilervorrichtung ist nach einer weiteren vorteilhaften Ausgestaltung der Erfindung im wesentlichen nach Art eines Dreiwegehahns ausgebildet und das Küken mittels eines Handriegels oder dergleichen Betätigungsmittel in die jeweilige Funktionsstellung überführbar. Von Vorteil ist das Küken drehbar in dem Gehäuse der Absperrverteilervorrichtung gelagert und es weist das Betätigungsmittel, sofern es handbetätigt ist, einen Handriegel in Form eines T-Stücks oder Y-Stückes auf.

Weiterhin hat es sich als vorteilhaft erwiesen, daß dem Betätigungsmittel, beispielsweise dem Handriegel oder dergleichen, ein an der Absperrverteilervorrichtung angeordneter Anschlag zugeordnet ist, der dem Verschwenkwinkel des Handriegels auf einen Winkel von bevorzugt ca. 180° oder auch etwas darunter begrenzt. Ist der Handriegel als T-Stück ausgebildet, so können die Endstücke des im wesentlichen auf der Drehachse des Kükens angeordneten freien Enden des T-Querschenkels gegen den Anschlag anschlagen und somit den Verschwenkwinkel auf ca. 180° oder etwas weniger begrenzen. Der T-Längsschenkel ist jedoch derart ausgebildet, daß dieser über den Anschlag hinweg verschwenkbar ist, wodurch insbesondere eine Zwischenstellung des Handriegels für den Anwender sichtbar gemacht oder verdeutlicht werden kann.

Dabei bietet es sich nach der Erfindung an, daß die erste oder dritte Funktionsstellung der Absperrverteilervorrichtung durch das in einer der beiden Winkelendlagen des Verschwenkwinkels positionierten Betätigungsmittels bestimmt ist. Im Falle der Ausgestaltung des Betätigungsmittels als T-Stück sind dies die Anschlagspunkte der freien Enden des T-Querschenkels an den Anschlag.

Von Vorteil ist die zweite Funktionsstellung der Absperrvorrichtung durch das in eine zwischen den beiden Winkelendlagen, bevorzugt in einer mittleren Winkellage positionierte, vorzugsweise in dieser Funktionsstellung verrastbare Betätigungsmittel bestimmt. Diese mittlere Winkellage kann beispielsweise dann eingenommen sein, wenn der T-Längsschenkel sich oberhalb des Anschlages befindet.

Von besonderem Vorteil ist nach einer anderen Ausgestaltung der Erfindung der erste Behälter mit Nahrung und der zweite Behälter mit Flüssigkeit gefüllt. Aufgrund dieser Maßnahme ist dafür Sorge getragen, daß vor einem gänzlichen Sperren der Strömungsverbindung von erstem und zweiten Behälter zur Anschlußleitung zunächst von der Zufuhr von Nahrung auf die Zufuhr von Flüssigkeit übergegangen werden muß, bis anschließend die Sperrstellung erreicht wird. Hierdurch ist sichergestellt, daß bei zunächst eingestellter Zufuhr von Nahrung vor dem gänzlichen Sperren der Strömungsverbindung zunächst auf die Zufuhr von Flüssigkeit übergegangen werden muß, wodurch eine Zwangsdurchspülung der Absperrverteilervorrichtung mit Flüssigkeit vor dem gänzlichen Abschalten erreicht wird.

Nach einer weiteren vorteilhaften Ausgestaltung der Erfindung ist die Absperrverteilervorrichtung in Sperrstellung zum ersten Anschlußstutzen, an dem der Flüssigkeitsbehälter angeschlossen ist, geöffnet. Die Sperrstellung ist in der dritten Funktionsstellung verwirklicht, in welcher die Strömungsverbindung von erstem und zweitem Behälter zur Anschlußleitung unterbrochen ist. Zum zweiten Anschlußstutzen hin, an dem die zum Patienten führende Anschlußleitung angeschlossen ist, und zum Einsteckteil hin, das mit dem Nahrungsbehälter in Verbindung steht, ist die Absperrverteilervorrichtung geschlossen. Durch diese Maßnahme wird erreicht, daß in Sperrstellung für beide Behälter die Absperrverteilervorrichtung mit Flüssigkeit gefüllt ist. Ein Anlösen oder Anbacken von festen Substanzen der Nahrung, die durch die Zwangsspülung nicht entfernt werden konnten, ist somit verhindert.

Von besonderem Vorteil ist eine einzige Dosiervorrichtung, wie ein Überlaufsystem oder eine Pumpe oder dergleichen, an der Vorrichtung vorgesehen, die in der von der Absperrverteilervorrichtung zum Patienten führenden Anschlußleitung angeordnet ist. Aufgrund dieser Maßnahme wird die Dosierung der unterschiedlichen Inhalte der beiden Behälter mittels einer einzigen Dosiervorrichtung gewährleistet, so daß ein mitunter umständliches Einstellen von zwei Dosiervorrichtungen, die jeweils einem der Behälter zugeordnet sind, vermieden ist.

Nach einer anderen Weiterbildung der Erfindung ist die Absperrverteilervorrichtung beziehungsweise deren Betätigungsmittel manuell, elektromotorisch oder elektrisch gesteuert, in die verschiedenen Funktionsstellungen überführbar.

Nach einer anderen Ausgestaltung der Erfindung sind der erste Behälter über ein Einsteckteil der Absperrverteilervorrichtung, der zweite Behälter über eine Zufuhrleitung und einen ersten Anschlußstutzen und die Anschlußleitung über einen zweiten Anschlußstutzten, bevorzugt mit Druckbuchse und Dichtung, mit der Absperrverteilervorrichtung verbunden. Der zweite Anschlußstutzten kann beispielsweise ein Außengewinde aufweisen, so daß die Anschlußleitung, beispielsweise mittels einer Überwurfmutter sicher an der Absperrverteilervorrichtung zu befestigen ist.

Aus konstruktiver Sicht hat es sich als vorteilhaft erwiesen, das Einsteckteil und zweiter Anschlußstutzen im wesentlichen koaxial benachbart zueinander angeordnet sind und der erste Anschlußstutzen mit dem zweiten Anschlußstutzen einen Winkel, beispielsweise von etwa 60°, einschließt. Die Kanäle des Einsteckteils sowie der beiden Anschlußstutzen treffen sich in einem mittleren Bereich des Gehäuses der Absperrverteilervorrichtung, in dem das Küken drehgelagert aufgenommen ist. Insgesamt erinnert die Positionierung von Einsteckteil und Anschlußstücken der Abstellverteilervorrichtung in einer Draufsicht einer Y-Form.

Von besonderem Vorteil weisen die mit dem Inhalt der Behälter in Kontakt kommenden Wandungen der Absperrverteilervorrichtung, insbesondere deren Kanalinnenwandungen im Bereich des Einsteckteils, des ersten und zweiten Anschlußstutzens sowie des Kükens beziehungsweise der topfförmigen Aufnahme für das Küken Toträume nicht auf. Insoweit ist dafür Sorge getragen, daß beim Einstecken beziehungsweise Anschließen des Zufuhrleitungen beziehungsweise Anschlußleitungen beziehungsweise der Behälter keine Toträume auftreten, in welchen sich Bakterien oder dergleichen bilden könnten.

Schließlich ist nach der Erfindung vorgesehen, daß die Vorrichtung vorzugsweise integraler Bestandteil des Überleitsystems, beispielsweise der Anschlußleitung ist. So kann das Ende der Vorrichtung mit einem Außengewinde versehen und als Schlauchsitz geformt sein.

Weitere Ziele, Vorteile, Merkmale und Anwendungsmöglichkeiten der vorliegenden Erfindung ergeben sich aus der nachfolgenden Beschreibung eines Ausführungsbeispieles anhand der Zeichnung. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Ansprüchen oder deren Rückbeziehung.

Es zeigen:
- Figur 1: eine Ansicht eines Ausführungsbeispiels einer erfindungsgemäßen Vorrichtung zur Flüssigkeitsbilanzierung bei enteral ernährten Patienten,
- Figur 2: eine perspektivische Darstellung eines Ausführungsbeispiels einer Absperrverteilervorrichtung,
- Figur 3: eine Seitenansicht der Absperrverteilervorrichtung der Figur 2,
- Figur 4: einen Schnitt durch die Absperrverteilervorrichtung der Figur 3 gemäß der Schnittlinie F-F,
- Figur 5: eine Draufsicht auf die Absperrverteilervorrichtung der Figur 2,
- Figur 6: einen Schnitt durch die Absperrverteilervorrichtung der Figur 8 gemäß der Schnittlinie E-E,
- Figur 7: einen Schnitt durch die Absperrverteilervorrichtung der Figur 3 gemäß Schnittlinie D-D,
- Figur 8: eine Draufsicht auf die Absperrverteilervorrichtung der Figur 2 in axialer Richtung mit Blick auf das Einsteckteil und
- Figur 9: Absperrverteilervorrichtung gemäß Figur 4, jedoch ohne Einsteckteil, in Sperrstellung.

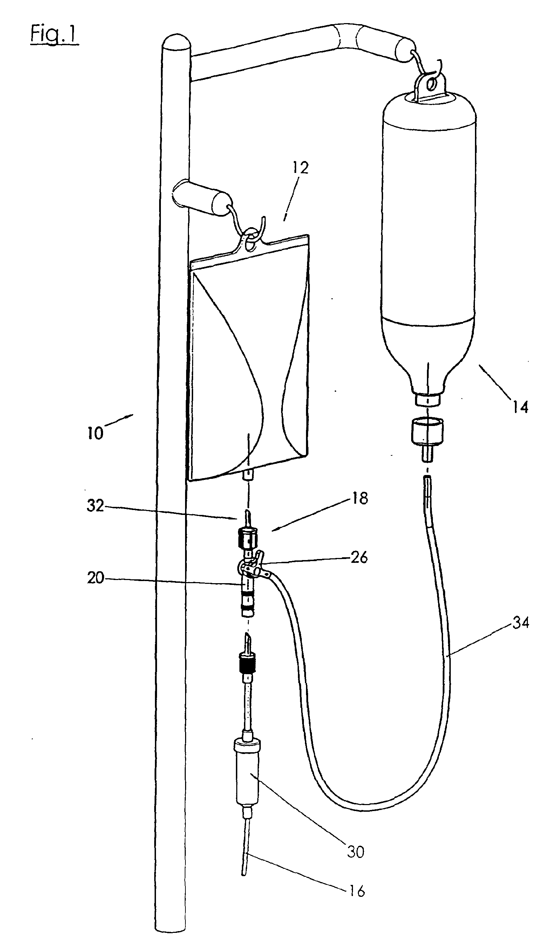

Die in Figur 1 dargestellte Vorrichtung 10 zur Flüssigkeitsbilanzierung bei enteral ernährten Patienten weist einen ersten Behälter 12 und einen zweiten Behälter 14 auf. Diese Behälter 12, 14 sind jeweils mit einer zum Patienten führenden Anschlußleitung 16 über ein Anschlußstück 18 verbunden. Die Behälter 12, 14 sind mit unterschiedlichen Inhalten, wie zum Beispiel unterschiedlicher Nahrung, unterschiedlichen Flüssigkeiten oder auch Nahrung beziehungsweise Flüssigkeit oder dergleichen, gefüllt. Das Anschlußstück 18 ist als Absperrverteilervorrichtung 20 mit mehreren Funktionsstellungen ausgebildet. Über diese Absperrverteilervorrichtung 20 kann die Strömungsverbindung zwischen dem ersten und/oder zweiten Behälter 12, 14 und der Anschlußleitung 16 wahlweise hergestellt beziehungsweise unterbrochen werden.

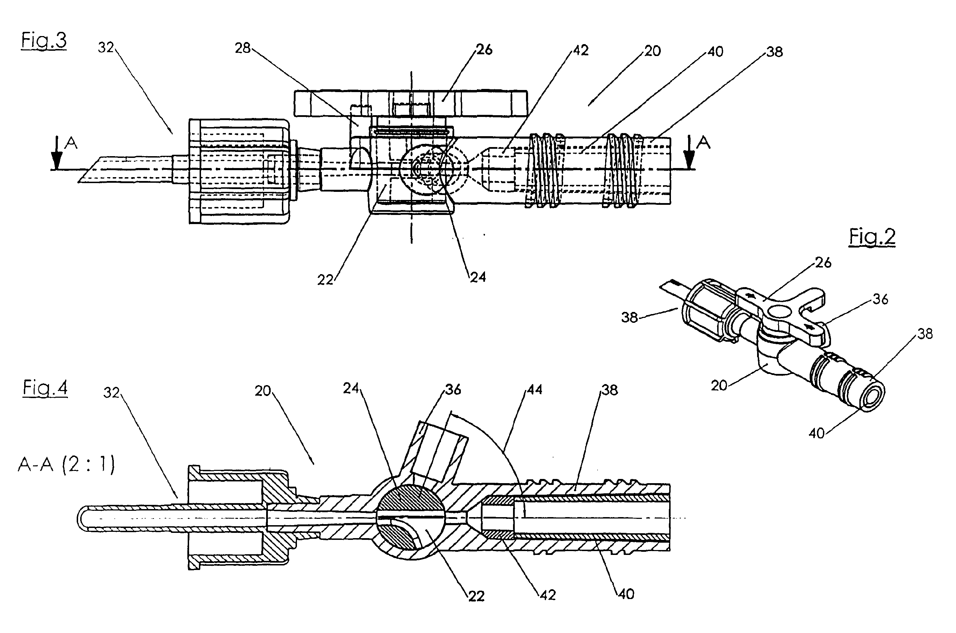

Insbesondere weist die Absperrverteilervorrichtung 20 drei Funktionsstellungen auf, wobei in einer ersten Funktionsstellung, die deutlich beispielsweise aus Figur 2, 4 und 5 ersichtlich ist, nur der erste Behälter 12 in Strömungsverbindung mit der Anschlußleitung 16 steht. In einer zweiten Funktionsstellung, bei der das Betätigungsmittel 26 der Absperrverteilervorrichtung 20 gegen den Uhrzeigersinn um ca. 90° oder etwas weniger verstellt ist, steht nur der zweite Behälter 14 in Strömungsverbindung mit der Anschlußleitung 16. Schließlich ist eine dritte Funktionsstellung vorgesehen, bei der das Betätigungsmittel 26 gegenüber der ersten Funktionsstellung um ca. 180° oder etwas weniger gegen den Uhrzeigersinn verstellt ist. In dieser Stellung ist die Strömungsverbindung von erstem und zweiten Behälter 12, 14 zur Anschlußleitung 16 beziehungsweise dem zweiten Anschlußstutzen 38 unterbrochen. Die Funktionsweise der Absperrverteilervorrichtung 20 wird insbesondere durch die Schnittdarstellung der Figur 4 verdeutlicht.

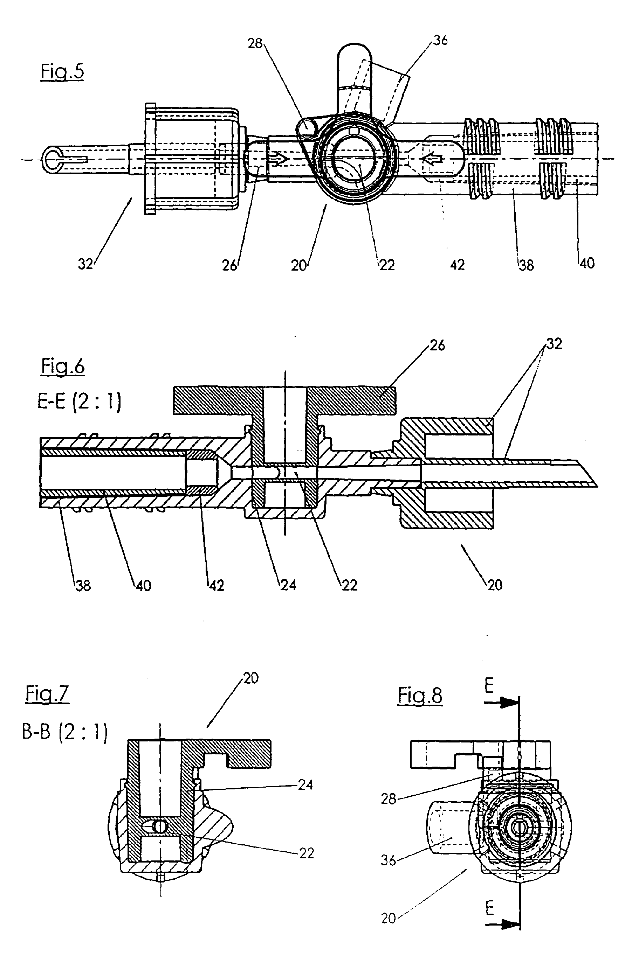

Die Absperrverteilervorrichtung 20 ist im wesentlichen nach Art eines Dreiwegehahns 22 ausgebildet, wobei das Küken 24 des Hahns mittels eines Handriegels oder dergleichen Betätigungsmittel 26 in die jeweilige Funktionsstellung überführbar. Weiterhin ist dem Betätigungsmittel 26, zum Beispiel dem Handriegel oder dergleichen, ein an der Absperrverteilervorrichtung 20 angeordneter Anschlag 28 zugeordnet, wie dies besonders deutlich aus Figur 5 ersichtlich ist. Der Anschlag 28 begrenzt den Verschwenkwinkel des Betätigungsmittels 26 auf einen Winkelbereich von bevorzugt etwa 180° oder etwas weniger. Dabei ist die erste und dritte Funktionsstellung der Absperrverteilervorrichtung 20 durch das in einer der beiden Winkelendlagen des Verschwenkwinkels positionierte Betätigungsmittel 26 bestimmt.

Die zweite Funktionsstellung der Absperrverteilervorrichtung 20 ist durch das in einer zwischen den beiden Winkelendlagen, bevorzugt in einer mittleren Winkellage positionierte, und in dieser Stellung verrastbare Betätigungsmittel 26 definiert.

Obwohl in den beiden Behältern grundsätzlich unterschiedliche Inhalte aufgenommen sein können, bietet es sich von Vorteil an, daß der erste Behälter 12 mit Nahrung und der zweite Behälter 14 mit Flüssigkeit gefüllt ist, so daß vor einem gänzlichen Sperren der Strömungsverbindung zwischen den beiden Behältern 12 und 14 und der Anschlußleitung 16 auf jeden Fall eine Strömungsverbindung zwischen den zweiten, mit Flüssigkeit gefüllten Behälter 14 und der Anschlußleitung 16 geschaffen wird, so daß eine Zwangsdurchspülung mit Flüssigkeit vor dem gänzlichen Abschalten der Vorrichtung stattfindet.

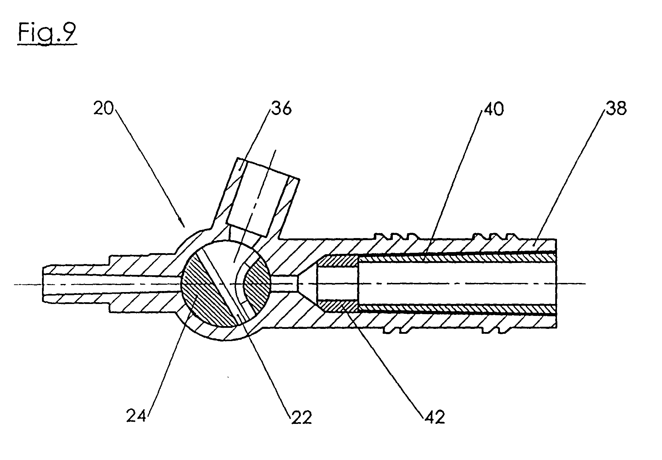

In Figur 9 ist die dritte Funktionsstellung der Absperrverteilervorrichtung 20 dargestellt, welche zum ersten Anschlußstutzen 36, an dem der mit Flüssigkeit gefüllte zweite Behälter 14 angeschlossen ist, geöffnet ist. Zum zweiten Anschlußstutzen 38 hin, an dem die zum Patienten führende Anschlußleitung 16 angebracht ist, sowie dem Einsteckteil 32 hin, welches mit den mit Nahrung gefüllten ersten Behälter 12 verbunden ist, ist die Absperrverteilervorrichtung 20 dagegen geschlossen. Damit ist die Absperrverteilervorrichtung 20 in ihrer Sperrstellung immer mit Flüssigkeit gefüllt, so daß ein Anlösen oder Anbacken von in der Nahrung enthaltenen festen Substanzen, die durch die Zwangsspülung nicht entfernt werden konnten, verhindert ist.

Wie aus Figur 1 ersichtlich ist, weist die Vorrichtung 10 eine einzige Dosiervorrichtung 30, wie ein Überlaufsystem oder auch eine Pumpe oder dergleichen auf, wobei die Dosiervorrichtung 30 in der von der Absperrverteilervorrichtung 20 zum Patienten führenden Anschlußleitung 16 angeordnet ist.

Die Absperrverteilervorrichtung 20 beziehungsweise deren Betätigungsmittel 26 kann manuell, elektromotorisch oder auch elektronisch gesteuert in die unterschiedlichen Funktionsstellungen überführt werden.

Der erste Behälter 12 ist über ein Einsteckteil 32 der Absperrverteilervorrichtung 20 mit dieser verbunden, während der zweite Behälter 14 über eine Zufuhrleitung 34 und einen ersten Anschlußstutzen 36 mit der Absperrverteilervorrichtung 20 in Verbindung steht. Die Anschlußleitung 16 ist hingegen über einen zweiten Anschlußstutzen 38, bevorzugt mit Druckbuchse 40 und Dichtung 42, an die Absperrverteilervorrichtung 20 angeschlossen. Das Einsteckteil 32 und der zweite Anschlußstutzen 38 sind im wesentlichen koaxial benachbart zueinander angeordnet, wobei der erste Anschlußstutzen 36 mit dem zweiten Anschlußstutzen 38 einen Winkel 44 von ungefähr 60° einschließt. Die mit dem Inhalt der Behälter 12, 14 in Kontakt kommenden Wandungen der Absperrverteilervorrichtung 20, insbesondere deren Innenwandung im Bereich des Einsteckteils 32, des ersten und zweiten Anschlußstutzens 36, 38 sowie des Kükens 24 weisen keine Toträume auf. Insoweit wird der Gefahr einer Bakterienbildung in etwaigen Toträumen entgegengewirkt.

### Bezugszeichenliste

- 10 -: Vorrichtung
- 12 -: erster Behälter
- 14 -: zweiter Behälter
- 16 -: Anschlußleitung
- 18 -: Anschlußstück
- 20 -: Absperrverteilervorrichtung
- 22 -: Dreiwegehahn
- 24 -: Küken
- 26 -: Betätigungsmittel
- 28 -: Anschlag
- 30 -: Dosiervorrichtung
- 32 -: Einsteckteil
- 34 -: Zufahrleitung
- 36 -: erster Anschlußstutzen
- 38 -: zweiter Anschlußstutzen
- 40 -: Druckbuchse
- 42 -: Dichtung
- 44 -: Winkel

## Patentansprüche

1. Vorrichtung (10) zur Flüssigkeitsbilanzierung bei enteral ernährten Patienten mit einem ersten Behälter (12) und einem zweiten Behälter (14), die jeweils mit einer zum Patienten führenden Anschlußleitung (16) über ein Anschlußstück (18) in Verbindung stehen, wobei die Behälter (12, 14) bevorzugt unterschiedliche Inhalte, wie Nahrung und Flüssigkeit, unterschiedliche Flüssigkeiten oder dergleichen, enthalten, **dadurch gekennzeichnet**, daß das Anschlußstück (18) als Absperrverteilervorrichtung (20) mit mehreren Funktionsstellungen ausgebildet ist, mit der die Strömungsverbindung zwischen dem ersten und/oder zweiten Behälter (12, 14) und der Anschlußleitung (16) wahlweise hergestellt und unterbrochen wird.

2. Vorrichtung nach Anspruch 1, **dadurch gekennzeichnet**, daß die Absperrverteilervorrichtung (20) drei Funktionsstellungen aufweist, wobei in einer ersten Funktionsstellung nur der erste Behälter (12) in Strömungsverbindung und in einer zweiten Funktionsstellung nur der zweite Behälter (14) in Strömungsverbindung mit der Anschlußleitung (16) stehen und in einer dritten Funktionsstellung die Strömungsverbindung von erstem und zweiten Behälter (12, 14) zur Anschlußleitung (16) unterbrochen ist.

3. Vorrichtung nach einem der vorhergehenden Ansprüche, **dadurch gekennzeichnet**, daß die Absperrverteilervorrichtung (20) im wesentlichen nach Art eines Dreiwegehahns (22) ausgebildet und das Küken (24) mittels eines Handriegels oder dergleichen Betätigungsmittel (26) in die jeweilige Funktionsstellung überführbar ist.

4. Vorrichtung nach einem der vorhergehenden Ansprüche, **dadurch gekennzeichnet**, daß dem Betätigungsmittel (26), zum Beispiel dem Handriegel oder dergleichen, ein an der Absperrverteilervorrichtung (20) angeordneter Anschlag (28) zugeordnet ist, der den Verschwenkwinkel des Betätigungsmittels (26) auf einen Winkelbereich von bevorzugt ca. 180° oder auch darunter begrenzt.

5. Vorrichtung nach Anspruch 4, **dadurch gekennzeichnet**, daß die erste oder dritte Funktionsstellung der Absperrverteilervorrichtung (20) durch das in einer der beiden Winkelendlagen des Verschwenkwinkels positionierten Betätigungsmittels (26) bestimmt ist.

6. Vorrichtung nach Anspruch 4 oder 5, **dadurch gekennzeichnet**, daß die zweite Funktionsstellung der Absperrvorrichtung (20) durch das in einer zwischen den beiden Winkelendlagen, bevorzugt in einer mittleren Winkellage positionierte, vorzugsweise in dieser Funktionsstellung verrastbare Betätigungsmittel (26) bestimmt ist.

7. Vorrichtung nach einem der vorhergehenden Ansprüche, **dadurch gekennzeichnet**, daß der erste Behälter (12) mit Nahrung und der zweite Behälter (14) mit Flüssigkeit gefüllt ist.

8. Vorrichtung nach Anspruch 7, **dadurch gekennzeichnet**, daß in der dritten Funktionsstellung, in welcher die Strömungsverbindung von erstem und zweitem Behälter (12, 14) zur Anschlußleitung (16) unterbrochen ist, die Absperrverteilervorrichtung (20) zum ersten Anschlußstutzen (36) geöffnet und zum zweiten Anschlußstutzen (38) sowie zum Einsteckteil (32) geschlossen ist.

9. Vorrichtung nach einem der vorhergehenden Ansprüche, **dadurch gekennzeichnet**, daß eine einzige Dosiervorrichtung (30), wie ein Überleitsystem oder eine Pumpe oder dergleichen, an der Vorrichtung (10) vorgesehen und in der von der Absperrverteilervorrichtung (20) zum Patienten führenden Anschlußleitung (16) angeordnet ist.

10. Vorrichtung nach einem der vorhergehenden Ansprüche, **dadurch gekennzeichnet**, daß die Absperrverteilervorrichtung (20) beziehungsweise deren Betätigungsmittel manuell, elektromotorisch oder elektrisch gesteuert in die verschiedenen Funktionsstellungen überführbar ist.

11. Vorrichtung nach einem der vorhergehenden Ansprüche, **dadurch gekennzeichnet**, daß der erste Behälter (12) über ein Einsteckteil (32) an der Absperrverteilervorrichtung (20), der zweite Behälter (14) über eine Zufuhrleitung (34) und einen ersten Anschlußstutzen (36) an der Absperrverteilervorrichtung (20) und die Anschlußleitung (16) über einen zweiten Anschlußstutzten (38) an der Absperrverteilervorrichtung (20), bevorzugt mit Druckbuchse (40) und Dichtung (42), mit der Absperrverteilervorrichtung (20) verbunden ist.

12. Vorrichtung nach Anspruch 11, **dadurch gekennzeichnet**, daß das Einsteckteil (32) und der zweite Anschlußstutzen (38) im wesentlichen koaxial benachbart zueinander angeordnet sind und der erste Anschlußstutzen (36) mit dem zweiten Anschlußstutzen (38) einen Winkel (44), beispielsweise von etwa 60°, einschließt.

13. Vorrichtung nach einem der vorhergehenden Ansprüche, **dadurch gekennzeichnet**, daß die mit dem Inhalt der Behälter (12, 14) in Kontakt kommende Wandungen der Absperrverteilervorrichtung (20), insbesondere deren Innenwandungen im Bereich des Einsteckteils (32), des ersten und zweiten Anschlußstutzens (36, 38) sowie des Kükens (24) beziehungsweise dessen Aufnahme im wesentlichen totraumfrei ausgebildet sind.

14. Vorrichtung nach einem der vorhergehenden Ansprüche, **dadurch gekennzeichnet**, daß sie vorzugsweise integraler Bestandteil des Überleitsystems, beispielsweise der Anschlußleitung (16) ist.
